(19) Europäisches Patentamt
European Patent Office
Office européen des brevets

(11) Veröffentlichungsnummer: **0 009 088**
**A1**

(12) # EUROPÄISCHE PATENTANMELDUNG

(21) Anmeldenummer: **79102514.1**

(22) Anmeldetag: **18.07.79**

(51) Int. Cl.³: **G 01 N 33/54**, C 07 F 9/09

(30) Priorität: **18.09.78 CH 9719/78**

(43) Veröffentlichungstag der Anmeldung: **02.04.80 Patentblatt 80/7**

(84) Benannte Vertragsstaaten: **CH DE FR GB IT NL**

(71) Anmelder: **F. Hoffmann-La Roche & Co. Aktiengesellschaft, Abt. RPM/P, CH-4002 Basel (CH)**

(72) Erfinder: **Barner, Richard, Dr., In den Reben 97, CH-4108 Witterswil (CH)**
Erfinder: **Boguth, Walter, Prof.,Dr., Sonneggstrasse 17, CH-4125 Riehen (CH)**

(74) Vertreter: **Kloter, Rudolf et al, Grenzacherstrasse 124 Postfach 601, CH-4002 Basel (CH)**

(54) **Reagenz zur Bestimmung von Wassermann-Antikörpern, Verfahren zu dessen Herstellung, Anwendung und Verwendung dieses Reagenzes zur Bestimmung von Wassermann-Antikörpern sowie entsprechende Reagenziengarnitur.**

(57) Es wird die Verwendung von Verbindungen der allgemeinen Formel

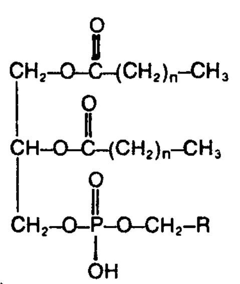

I

worin
n eine ganze Zahl von 12 bis 16 ist und
R Phenyl oder substituiertes Phenyl bedeutet,
und/oder von Salzen davon zur Bestimmung von Wassermann-Antikörpern beschrieben. Neben der Herstellung dieser Verbindungen wird angegeben, wie die Wassermann-Antikörper mittels der Verbindungen der allgemeinen Formel I und/oder deren Salzen bestimmt werden können. Schließlich wird auch eine entsprechende Reagenzgarnitur enthaltend eine Verbindung der Formel I und/oder ein Salz davon offenbart.

EP 0 009 088 A1

0009088

F. Hoffmann-La Roche & Co. Aktiengesellschaft, Basel, Schweiz

RAN 4093/046

Vorklassifizierungsnummer C.P.79/003188

Reagens zur Bestimmung von Wassermann-Antikörpern, Verfahren zu dessen Herstellung, Anwendung und Verwendung dieses Reagens zur Bestimmung von Wassermann-Antikörpern sowie entsprechende Reagentiengarnitur.

Die vorliegende Erfindung betrifft ein neues Reagenz zur Bestimmung von Wassermann-Antikörpern, enthaltend ein Phosphatidsäurederivat mit Cardiolipinaktivität.

Die Bestimmung von Wassermann-Antikörpern in physiologischen Flüssigkeiten, hauptsächlich im Blutserum, dient zum Nachweis von Syphilis und wird, insbesondere für die Kontrolle von Blut und Blutkonserven für Transfusionen, in grösserem Massstab durchgeführt.

Es ist bekannt, dass man Cardiolipin, ein aus Rinderherzmuskel extrahiertes Phospholipid der allgemeinen Formel

 

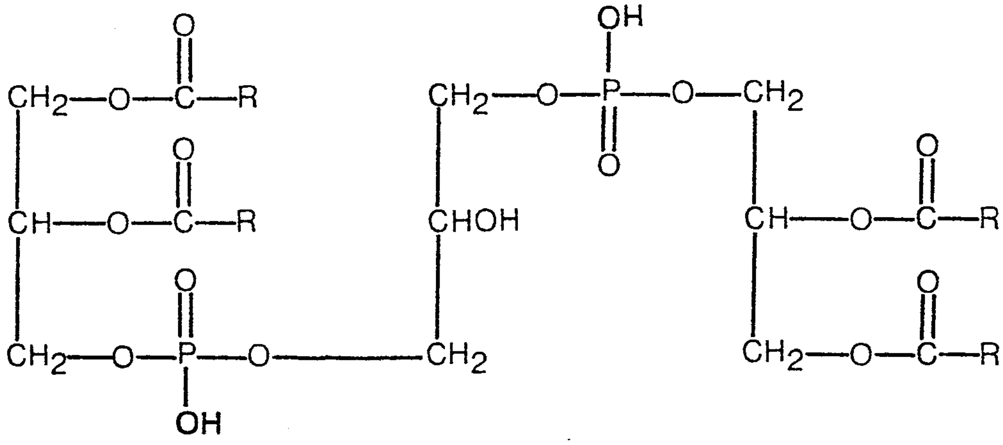

wobei RCO- einen ungesättigten Fettsäurerest bedeutet, als Hapten für den serologischen Nachweis von Wassermann-Antikörpern verwenden kann.

Die Versuche, Cardiolipin durch ein auf synthetischem Wege leicht zugängliches Analogon mit einfacherer Struktur zu ersetzen, haben bisher zu keinen befriedigenden Ergebnissen geführt. Die bis anhin synthetisierten Cardiolipinanaloga zeigen im serologischen Syphilistest keine oder nur eine im Verhältnis zu Cardiolipin sehr schwache Wirkung (vgl. z.B. Nature, 206 (1965) Seite 135).

Es wurde nun gefunden, dass Verbindungen der allgemeinen Formel

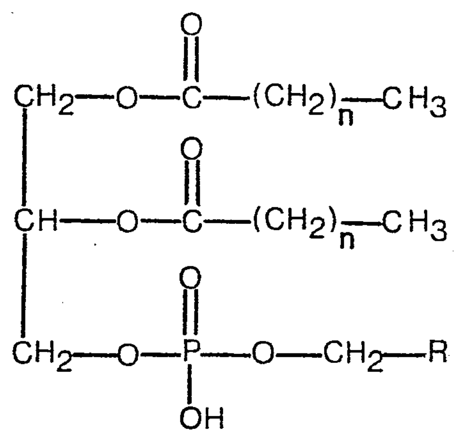

I

worin n eine ganze Zahl von 12 bis 16 ist und
R Phenyl oder substituiertes Phenyl bedeutet,
und/oder deren Salze, eine ähnliche Aktivität wie das natürliche Cardiolipin besitzen.

Der Ausdruck "substituiertes Phenyl" bezeichnet diese Gruppe, wenn sie mit einem oder mehreren der folgenden Substituenten substituiert ist: Halogen, d.h. Fluor, Chlor, Brom oder Jod, niederes Alkyl, Trifluormethyl, niederes Alkoxy, Nitro und Cyano.

Als Salze der Verbindungen I eignen sich alle wasserlöslichen Salze, insbesondere die Alkalisalze, wie das Natrium- und das Kaliumsalz; die Erdalkalisalze, wie das Calciumsalz, Ammonium- und substituierte Ammoniumsalze, das Magnesium- und das Bariumsalz.

Die Verbindungen der Formel I besitzen eine wesentlich einfachere Struktur als Cardiolipin. Sie sind auf synthetischem Weg leicht zugänglich und können als Racemate verwendet werden. Ein weiterer Vorteil der Verbindungen der Formel I ist ihre gute Löslichkeit und die hohe Stabilität der entsprechenden Lösungen. Ferner können die Verbindungen der Formel I durch Kristallisation leicht in reiner Form hergestellt werden. Schliesslich besitzen sie gegenüber Cardiolipin auch noch den Vorteil, dass sie bei Raumtemperatur ohne Schutzmassnahmen lagerfähig sind, während Cardiolipin im Kühlschrank unter Stickstoffatmosphäre aufbewahrt werden muss.

Bevorzugte Verbindungen der Formel I sind diejenigen, worin n 14 ist und R für Phenyl steht, insbesondere

D,L-(2,3-Di-O-palmitoylglycerol)benzylphosphorsäure

Natrium-D,L-(2,3-di-O-palmitoylglycerol)benzylphosphorsäure

Kalium-D,L-(2,3-di-O-palmitoylglycerol)benzylphosphorsäure

Calcium-D,L-(2,3-di-O-palmitoylglycerol)benzylphosphorsäure

Magnesium-D,L-(2,3-di-O-palmitoylglycerol)benzylphosphorsäure.

Besonders bevorzugt ist Natrium-D,L-(2,3-di-O-palmitoylglycerol)benzylphosphorsäure. Diese Verbindung wird in Chem. Pharm. Bull (Tokyo) 11, 1150 (1963) als Zwischenprodukt für die Herstellung von Cardiolipinanaloga beschrieben. Diese Literaturstelle enthält jedoch keinen Hinweis über die Reaktionsfähigkeit dieser Verbindung mit Wassermann-Antikörpern und ihrer möglichen Anwendung in einem Syphilistest.

Die Verbindungen der Formel I können in an sich bekannter Weise nach folgendem Reaktionsschema erhalten werden:

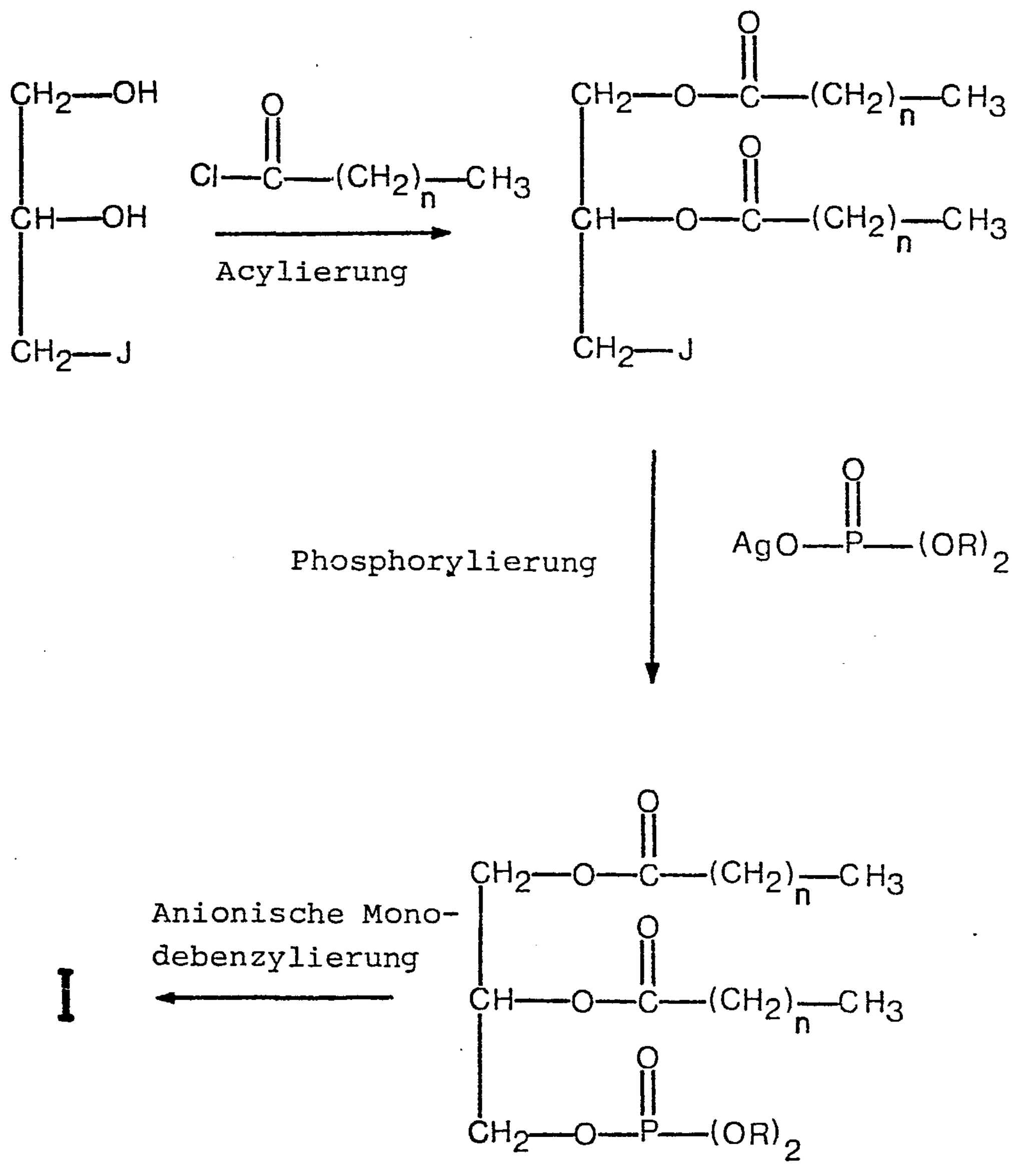

Die Verbindungen der Formel I dienen zur Herstellung von Reagenzien zur Bestimmung von Wassermann-Antikörpern. Diese Reagenzien können hergestellt werden, indem man eine Verbindung der allgemeinen Formel I mit einem geeigneten Trägermaterial vermischt. Falls das Trägermaterial ein Protein ist, wird die Verbindung der allgemeinen Formel I vorzugsweise chemisch daran gekuppelt.

Ein für die Zwecke der vorliegenden Erfindung besonders geeignetes Trägermaterial ist jedoch eine Mischung eines Lecithins, vorzugsweise Eilecithin und Cholesterin. In diesem Fall wird das erhaltene Reagenz, welches einfach aus einer Mischung enthaltend eine Verbindung der Formel I, ein Lecithin und Cholesterin besteht, direkt ohne chemische Bearbeitung, zur Bestimmung von Wassermann-Antikörpern eingesetzt.

Zur Bestimmung von Wassermann-Antikörpern werden die erfindungsgemässen Reagenzien mit der Probe, vorzugsweise Serumprobe, vermischt und die Intensität der immunologischen Reaktion gemessen.

Das Mengenverhältnis der Probe zum Reagenz kann variieren, liegt jedoch mit Vorteil zwischen 1 zu 1 und 5 zu 1, vorzugsweise bei 5 zu 2.

Falls das erfindungsgemässe Reagenz aus einer Mischung enthaltend eine Verbindung der Formel I, ein Lecithin und Cholesterin besteht, erfolgt die Bestimmung, indem man

a) die Verbindung der Formel I, Lecithin und Cholesterin vermischt,

b) die Probe zugibt und

c) das Ausmass der Flockung beobachtet.

Stufe a) erfolgt mit Vorteil in Gegenwart eines wassermischbaren inerten organischen Lösungsmittels, vorzugsweise eines niederen Alkohols wie Aethanol, oder Tetrahydrofuran und eines physiologischen Kochsalzpuffers. Es entsteht eine milchige Suspension mit Teilchen, die einige Mikron gross sind. In der in Stufe a) hergestellten Mischung liegt die Verbindung der Formel I vorzugsweise in einer Konzentration von 0,02 bis 0,20 mg/ml vor, wobei eine Konzentration von 0,05 bis 0,10 mg/ml besonders bevorzugt wird. Die Konzentration an Lecithin in dieser Mischung liegt mit Vorteil zwischen 0,15 und 0,3 mg/ml, vorzugsweise bei 0,25 mg/ml. Die Konzentration an Cholesterin in dieser Mischung liegt mit Vorteil zwischen 0,8 bis 1,2 mg/ml, vorzugsweise bei 0,9 mg/ml. Der Volumenanteil an physiologischer Kochsalzlösung in dieser Mischung liegt vorzugsweise zwischen 85% und 95%, wobei 90% besonders bevorzugt ist. Der Volumenanteil an inertem organischen Lösungsmittel in dieser Mischung liegt mit Vorteil zwischen 5% und 15%, vorzugsweise bei 10%.

Falls die in Stufe b) zugegebene Probe Wassermann-Antikörper enthält, erfolgt eine Flockung, wobei das Ausmass der Flockung von der Menge an Wassermann-Antikörpern abhängt.

Die Messung des Ausmasses der Flockung erfolgt bevorzugt durch Ablesung der Flockengrösse unter dem Mikroskop, 2 bis 10 Minuten nach Zugabe der Probe.

Die erfindungsgemässen Verbindungen der Formel I können als Lösungen z.B. als wässrige äthanolische Lösung oder in fester Form, z.B. als kristallines Pulver aufbewahrt werden. Sie werden bevorzugt in fester Form in einem Behälter einer Reagenzgarnitur, welche in weiteren Behältern das Trägermaterial, vorzugsweise Lecithin und Cholesterin und/oder Kontrollserum enthalten kann, verpackt.

Die Erfindung wird anhand des nachstehenden Beispiels veranschaulicht.

Beispiel 1

Es wird eine äthanolische Mischlösung von Natrium-D,L-(2,3-di-O-palmitoylglycerol)benzylphosphorsäure (1,0 mg/ml), Eilecithin (2,5 mg/ml) und Cholesterin (9,0 mg/ ml) im Verhältnis 1:9 mit einem physiologischen Kochsalzpuffer vermischt. Es entsteht eine milchige Suspension mit Teilchen, die einige µm gross sind. 20 µl dieses Reagenzes werden mit 50 µl Serum zusammengerührt und 4 Minuten lang auf der Testplatte geschaukelt. Mit Syphilis-positivem Serum aggregieren die Teilchen. Die Ablesung der Flockengrösse erfolgt unter dem Mikroskop (50 x) und ermöglicht einen semi-quantitativen Nachweis von Syphilis.

## Patentansprüche

1. Reagenz zur Bestimmung von Wassermann-Antikörpern, enthaltend eine Verbindung der allgemeinen Formel

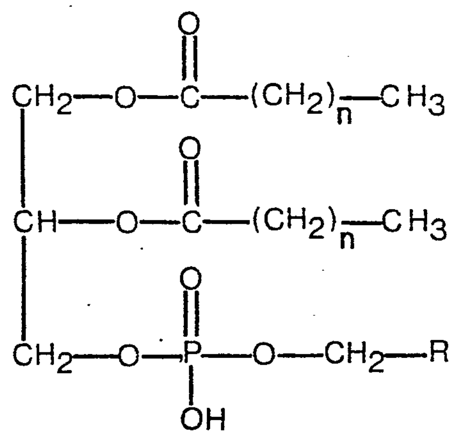

I

worin n eine ganze Zahl von 12 bis 16 ist und R Phenyl oder substituiertes Phenyl bedeutet, und/oder deren Salze und ein Trägermaterial.

2. Reagenz nach Anspruch 1, dadurch gekennzeichnet, dass die Verbindung der Formel I Natrium-D,L-(2,3-di-O-palmitoylglycerol)benzylphosphorsäure ist.

3. Reagenz nach Anspruch 1 oder 2, dadurch gekennzeichnet, dass das Trägermaterial eine Mischung von Lecithin und Cholesterin ist.

4. Reagenz nach Anspruch 1, dadurch gekennzeichnet, dass das Lecithin Eilecithin ist.

5. Reagenz nach einem der Ansprüche 1 bis 4, dadurch gekennzeichnet, dass es einen niederen Alkohol enthält.

6. Reagenz nach Anspruch 5, dadurch gekennzeichnet, dass der niedere Alkohol Aethanol ist.

7. Reagenz nach einem der Ansprüche 1 bis 6, dadurch gekennzeichnet, dass es eine physiologische Kochsalzlösung enthält.

8. Reagenz nach einem der Ansprüche 1 bis 7, enthaltend die Verbindung der Formel I, das Lecithin und das Cholesterin in einer Konzentration von 0,02 bis 0,20 mg/ml bzw. 0,1 bis 0,3 mg/ml bzw. 0,8 bis 1,2 mg/ml.

9. Reagenz nach Anspruch 8, enthaltend die Verbindung der Formel I, das Lecithin und das Cholesterin in einer Konzentration von 0,05 bis 0,10 mg/ml bzw. 0,25 mg/ml bzw. 0,9 mg/ml.

10. Verfahren zur Herstellung eines Reagens nach einem der Ansprüche 1 bis 9, dadurch gekennzeichnet, dass man eine Verbindung der allgemeinen Formel

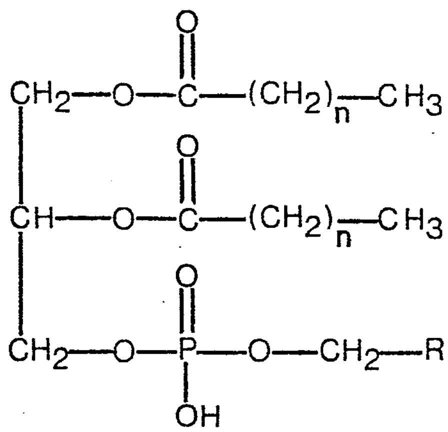

I

worin n eine ganze Zahl von 12 bis 16 ist und R Phenyl oder substituiertes Phenyl bedeutet, und/oder deren Salze mit einem Trägermaterial vermischt.

0009088

11. Verfahren zur Bestimmung von Wassermann-Antikörpern in einer Probe, dadurch gekennzeichnet, dass man ein Reagenz gemäss einem der Ansprüche 1 bis 9 mit der Probe vermischt und die Intensität der immunologischen Reaktion misst.

12. Verfahren nach Anspruch 11, dadurch gekennzeichnet, dass die Probe eine Serumprobe ist.

13. Verfahren nach einem der Ansprüche 11 und 12, dadurch gekennzeichnet, dass das Mengenverhältnis der Probe zum Reagenz 1 zu 1 bis 5 zu 1, vorzugsweise 5 zu 2 beträgt.

14. Verfahren nach einem der Ansprüche 11 bis 13, dadurch gekennzeichnet, dass man

a) eine Verbindung der Formel

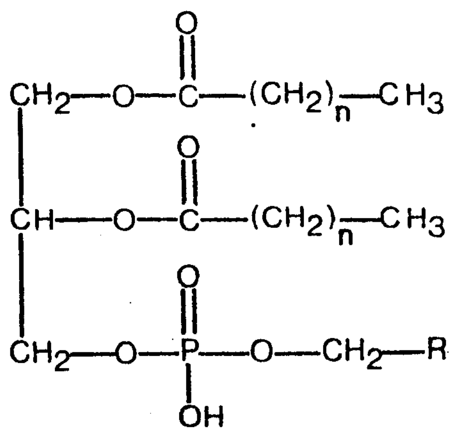

I

worin n eine ganze Zahl von 12 bis 16 ist und R Phenyl oder substituiertes Phenyl bedeutet,

und/oder deren Salze, Lecithin und Cholesterin vermischt,

b) Serumprobe zugibt und

c) das Ausmass der Flockung beobachtet.

15. Reagenziengarnitur zur Bestimmung von Wassermann-Antikörpern, enthaltend in einem Behälter eine Verbindung der allgemeinen Formel

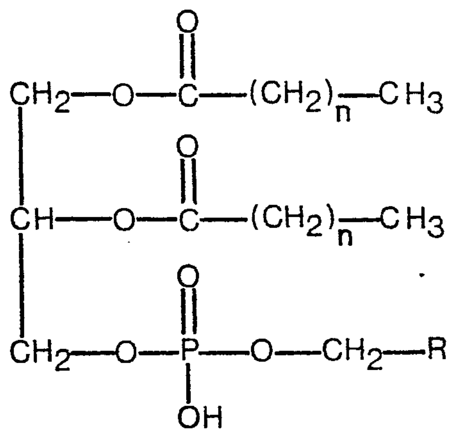

I

worin n eine ganze Zahl von 12 bis 16 ist und
R Phenyl oder substituiertes Phenyl bedeutet,
und/oder deren Salze und erwünschtenfalls in einem oder mehreren weiteren Behältern Trägermaterial und/oder Kontrollserum.

16. Reagenziengarnitur nach Anspruch 15, dadurch gekennzeichnet, dass die Verbindung der Formel I in fester Form vorliegt.

17. Reagenziengarnitur nach einem der Ansprüche 15 und 16, dadurch gekennzeichnet, dass das Trägermaterial Lecithin und Cholesterin ist.

18. Verwendung einer Verbindung der allgemeinen Formel

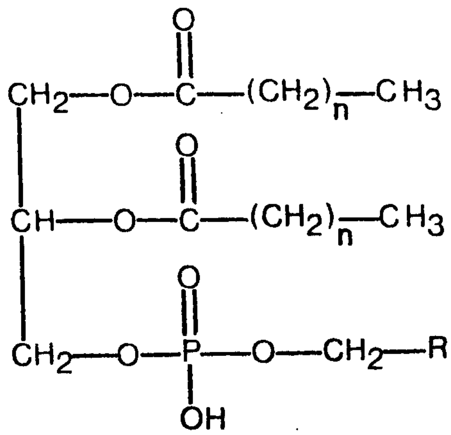

I ,

worin n eine ganze Zahl von 12 bis 16 ist und R Phenyl oder substituiertes Phenyl bedeutet, und/oder deren Salze zur Herstellung von Reagenzien, die zur Bestimmung von Wassermann-Antikörpern dienen.

***

Europäisches Patentamt

# EUROPÄISCHER RECHERCHENBERICHT

0009088

Nummer der Anmeldung

EP 79 102 514.1

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| A | DE - A1 - 2 711 288 (NIPPON CHEMIPHAR CO.)<br>* ganzes Dokument *<br>-- | | G 01 N 33/54<br>C 07 F 9/09 |
| A | FR - A2 - 2 349 836 (CENTRE REGIONAL DE TRANSFUSION SANGUINE DE LILLE)<br>* ganzes Dokument *<br>-- | | |
| A | FR - A1 - 2 266 169 (CENTRE REGIONAL DE TRANSFUSION SANGUINE DE LILLE)<br>* ganzes Dokument *<br>-- | | RECHERCHIERTE SACHGEBIETE (Int. Cl.³)<br>C 07 F 9/09<br>G 01 N 33/54 |
| A | Chemical Abstracts Band 59, Nr. 12, 9. Dezember 1963<br>Columbus, Ohio, USA<br>K. INOUE et al. "Cardiolipin analogs - syntheses of dipalmitoyl -DL-α-glyceryl-phosphorylpropanol sodium salt and bis(dipalmitoyl-DL-α-glycerylphosphoryl)-1,3-propanediol disodium salt"<br>Spalte 13814 b bis h | | |
| | & Chem. Pharm. Bull., Band 11, Nr. 9, 1963, Seiten 1150 bis 1156<br>-- ../.. | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 13-12-1979 | SCHWARTZ |

Europäisches Patentamt

**EUROPÄISCHER RECHERCHENBERICHT**

Nummer der Anmeldung
EP 79 102 514.1
- Seite 2 -

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | MÜNCHENER MEDIZINISCHE WOCHENSCHRIFT , Band 117, Nr. 23, 1975, München<br>G. POETSCHKE et al. "Moderne serologische Luesdiagnostik"<br>Seiten 999 bis 1004<br>* ganzes Dokument *<br>---- | | |
| | | | RECHERCHIERTE SACHGEBIETE (Int. Cl.3) |